# EUROPEAN PATENT APPLICATION

(11) **EP 4 269 434 A1**
(43) Date of publication of application: **01.11.2023**
(21) Application number: 21909453.9
(22) Date of filing: 22.12.2021
(51) Int. Cl.: C07K 16/28, A61K 39/395, C12N 15/13, A61P 35/00

(54) **ANTI-PD-L1 ANTIBODY AND USE THEREOF**

(30) Priority: 23.12.2020 CN 202011541107
(71) Applicant: Guangdong Fapon Biopharma Inc., Dongguan, Guangdong 523808 (CN)
(72) Inventor: LU, Lisheng, Dongguan, Guangdong 523808 (CN); HUO, Yongting, Dongguan, Guangdong 523808 (CN); ZHANG, Chan, Dongguan, Guangdong 523808 (CN); LUO, Ying, Dongguan, Guangdong 523808 (CN); LU, Di, Dongguan, Guangdong 523808 (CN); LIU, Yunpeng, Dongguan, Guangdong 523808 (CN); FU, Jun, Dongguan, Guangdong 523808 (CN)
(74) Representative: De Clercq & Partners
(86) International application number: PCT/CN2021/140391
(87) International publication number: WO 2022/135457

(57) **Abstract**

The present disclosure relates to the field of biomedicine, in particular to an anti-PD-L1 antibody and the use thereof. The anti-PD-L1 antibody or the antigen-binding fragment thereof provided in the present disclosure binds to a PD-L1 protein with a high affinity and high specificity, can effectively block the interaction between PD-L1 expressed on a cell surface and PD-1, and has the biological function activity of stimulating the production of cytokine, and has wide application prospects.

## Description

### Cross-Reference to Related Application

The present application claims priority to Chinese Patent Application No. 202011541107.9 filed to the China National Intellectual Property Administration on December 23, 2020 and entitled "Anti-PD-L1 Antibody and Use thereof", the disclosure of which is hereby incorporated by reference in its entirety.

### Technical Field

The application relates to the technical field of immunology, and specifically to an anti-PD-L1 antibody and use thereof.

### Background

A programmed cell death protein 1 (PD-1, also called CD279) is a costimulatory receptor expressed on the surface of an antigen-stimulated T cell. PD-L1 (CD274) and PD-L2 (CD273) are two ligands of the PD-1. The PD-L1 is expressed in hematopoietic cells including T cells, B cells, macrophages, dendritic cells and giant cells, and non-hematopoietic cells including vascular endothelial cells, keratinocytes, islet cells, astrocyte, placental syncytial trophoblast, corneal epidermis and endothelial cells. The PD-L1 and the PD-L2 are expressed in various tumor cells and tumor stroma.

Both the PD-1 and the PD-L1 belong to an I-shaped transmembrane protein in an immune superfamily protein, and are composed of Ig-V and Ig-C like extracellular domains, transmembrane domains and intracellular structural domains of short sequences. The interaction between a PD-L1 extracellular domain and a PD-1 extracellular domain can induce conformational changes in the PD-1 protein, such that an intracellular Immunoreceptor Tyrosine-based Inhibitory Motif (ITIM) and an Immunoreceptor Tyrosine-based Switch Motif (ITSM) are induced to be phosphorylated by Src family kinase. The phosphorylated tyrosine motif subsequently recruits SHP-2 and SHP-1 protein tyrosine phosphatases to downregulate a T-cell activation signal. In addition to the PD-1, the PD-L1 interacts with CD80, such that a signal of inhibiting the activity of the T cells is transmitted. The PD-1 interacts with the PD-L1 to downregulate the activity of the T cells in a plurality of aspects, including inhibition of the proliferation of the T cells, releasing of cytokine, and other effect functions.

The interaction between the PD-1 and the PD-L1 is essential for homeostasis of an immune system. PD-1-deficient mice of different genotypes tend to develop lupus-like autoimmune disease or fatal autoimmune cardiomyopathy. The PD-1-deficient mice alter the domestication of thymic T cells, and the blocking of the PD-L1 is able to disrupt tolerance between the fetus and the mother. In addition, inhibition of the interaction between the PD-1 and the PD-L1 enhances host immunity against pathogens.

The PD-L1 is expressed on various tumors (such as renal cancer, gastric cancer, urethral epithelial cancer, ovarian cancer and melanoma) with poor prognosis, such that with a PD-L1 antibody, tumor cells can be killed or the activity of killer T cells can be inhibited. In view of the importance of the PD-1 and the PD-L1 in down-regulation of immunoreaction, developing the anti-PD-L1 antibody for blocking the interaction between the PD-L1 protein and the PD-1 is of great significance for the use in tumor immunotherapy.

### Summary

The technical problem to be solved in this disclosure is to overcome the shortcomings and deficiencies of the low affinity and specificity of existing anti PD-L1 antibodies in binding to PD-L1, which cannot effectively block the binding of PD-L1 to PD-L1, and to provide an anti PD-L1 antibody and its application.

The application provides an anti-PD-L1 antibody or an antigen-binding fragment thereof. The antibody includes the following Complementarity Determining Regions (CDRs):
an HCDR1 as shown in an amino acid sequence, which is shown as any one of SEQ ID NO.19, 25, 31 or 37 or has at least 95% identity to a sequence as shown in any one of SEQ ID NO.19, 25, 31 or 37; an HCDR2 as shown in an amino acid sequence, which is shown as any one of SEQ ID NO.20, 26, 32 or 38 or has at least 95% identity to a sequence as shown in any one of SEQ ID NO.20, 26, 32 or 38; an HCDR3 as shown in an amino acid sequence, which is shown as any one of SEQ ID NO.21, 27, 33 or 39 or has at least 95% identity to a sequence as shown in any one of SEQ ID NO.21, 27, 33 or 39;
an LCDR1 as shown in an amino acid sequence, which is shown as any one of SEQ ID NO.22, 28, 34 or 40 or has at least 95% identity to a sequence as shown in any one of SEQ ID NO.22, 28, 34 or 40; an LCDR2 as shown in an amino acid sequence, which is shown as any one of SEQ ID NO.23, 29, 35 or 41 or has at least 95% identity to a sequence as shown in any one of SEQ ID NO.23, 29, 35 or 41; and an LCDR3 as shown in an amino acid sequence, which is shown as any one of SEQ ID NO.24, 30, 36 or 42 or has at least 95% identity to a sequence as shown in any one of SEQ ID NO.24, 30, 36 or 42.

The application provides a nucleic acid. The nucleic acid includes a first nucleic acid encoding a heavy chain variable region of the antibody or the antigen-binding fragment thereof, and/or a second nucleic acid encoding a light chain variable region of the antibody or the antigen-binding fragment thereof.

The application further provides a vector. The vector includes the nucleic acid.

The application further provides a cell. The cell includes the nucleic acid or the vector.

The application further provides a pharmaceutical composition. The pharmaceutical composition includes the anti-PD-L1 antibody or the antigen-binding fragment thereof, the nucleic acid, the vector or the cell.

The application further relates to the applications of the anti-PD-L1 antibody or the antigen-binding fragment thereof, and the nucleic acid, the vector, the cell or the pharmaceutical composition related to the anti-PD-L1 antibody or the antigen-binding fragment thereof in preparation of drugs for treating PD-L1-mediated diseases or symptoms.

### Brief Description of the Drawings

In order to illustrate the technical solutions in the embodiments of the application more clearly, the drawings used in the embodiments will be briefly introduced below. It is to be understood that the following drawings only illustrate some embodiments of the application, which therefore should not be regarded as limitations to the scope. For those of ordinary skill in the art, other related drawings may also be obtained in accordance with these drawings without creative efforts.
Fig. 1 shows a detection result of the binding activity of an anti-human PD-L1 antibody to a CHO empty cell.
Fig. 2 shows a detection result of the binding activity of an anti-human PD-L1 antibody to CHO-hPD-L1 stably expressing human PD-L1.
Fig. 3 shows a detection result of the binding activity of an anti-human PD-L1 antibody to CHO-cynoPD-L1 stably expressing macaque PD-L1.
Fig. 4 shows a detection result of the binding activity of an anti-human PD-L1 antibody to an IFNγ-stimulated A375 cell.
Fig. 5 shows a detection result of the competitive binding activity of an anti-human PD-L1 antibody to CHO-hPD-L1.
Fig. 6 shows a detection result of the binding activity of CHO-hPD-L1 to an hPD-L1 hFc recombinant protein blocked by an anti-human PD-L1 antibody in an experimental method 1.
Fig. 7 shows a detection result of a mode II of the binding activity of CHO-hPD-L1 to an hPD-L1 hFc recombinant protein blocked by an anti-human PD-L1 antibody in an experimental method 2.
Fig. 8 shows a detection result of the binding activity of CHO-hPD-L1 to an hCD80hFc recombinant protein blocked by an anti-human PD-L1 antibody.
Fig. 9 shows the in vitro activity results of anti-human PD-L1 antibodies analyzed by luciferase reporter gene method in plate I and plate II, wherein Fig. (a) shows a plate I result; and Fig. (b) shows a plate II result.
Fig. 10 shows the results of IL-2 secretion in mixed lymphocyte reaction mediated by anti-human PD-L1 antibody in Plate I.
Fig. 11 shows the results of IL-2 secretion in mixed lymphocyte reaction mediated by anti-human PD-L1 antibody in Plate II.
Fig. 12 shows the results of IFN-γ secretion in mixed lymphocyte reaction mediated by anti-human PD-L1 antibody in Plate I.
Fig. 13 shows the results of IFN-γ secretion in mixed lymphocyte reaction mediated by anti-human PD-L1 antibody in Plate II.
Fig. 14 shows results of the binding specificity of an anti-human PD-L1 antibody to recombinant proteins hPD-L1 hFc, hPD-L2 hFc, hICOSLGhFc and hB7-H3 hFc, wherein Fig. (a) shows a result of the binding specificity with the recombinant protein hPD-L1 hFc; Fig. (b) shows a result of the binding specificity with the recombinant protein hPD-L2 hFc; Fig. (c) shows a result of the binding specificity with the recombinant protein hICOSLGhFc; and Fig. (d) shows a result of the binding specificity with the recombinant protein hB7-H3 hFc.
Fig. 15 shows results of the binding specificity of an anti-human PD-L1 antibody to recombinant proteins hCD28 hFc, hCD86 hFc and hCTLA-4 hFc, wherein Fig. (a) shows a result of the binding specificity with the recombinant protein hCD28 hFc; Fig. (b) shows a result of the binding specificity with the recombinant protein hCD86 hFc; and Fig. (c) shows a result of the binding specificity with the recombinant protein hCTLA-4 hFc.
Fig. 16 shows a characterization result of in vivo activity of an anti-human PD-L1 antibody, wherein Fig. (a) shows a result of the impact of an mlgG1 subtype anti-hPD-L1 antibody on the tumor volume of a mouse; Fig. (b) shows a result of the impact of an mlgG2a subtype anti-hPD-L1 antibody on the tumor volume of the mouse; and Fig. (c) shows a result of the impact of the mlgG1 subtype anti-hPD-L1 antibody and the mlgG2a subtype anti-hPD-L1 antibody on the weight of the mouse.

### Detailed Description of the Embodiments

The application is further described below with reference to specific embodiments, but the embodiments do not limit the application in any form. Unless otherwise specified, reagents, methods and devices used in the application are conventional reagents, methods and devices in the present technical field.

Unless otherwise specified, the reagents and materials used in the following embodiments are commercially available.

Some implementations of the application disclose an anti-PD-L1 antibody or an antigen-binding fragment thereof. The antibody includes the following CDRs:
an HCDR1 as shown in an amino acid sequence, which is shown as any one of SEQ ID NO.19, 25, 31 or 37 or has at least 95% identity to a sequence as shown in any one of SEQ ID NO.19, 25, 31 or 37; an HCDR2 as shown in an amino acid sequence, which is shown as any one of SEQ ID NO.20, 26, 32 or 38 or has at least 95% identity to a sequence as shown in any one of SEQ ID NO.20, 26, 32 or 38; an HCDR3 as shown in an amino acid sequence, which is shown as any one of SEQ ID NO.21, 27, 33 or 39 or has at least 95% identity to a sequence as shown in any one of SEQ ID NO.21, 27, 33 or 39;
an LCDR1 as shown in an amino acid sequence, which is shown as any one of SEQ ID NO.22, 28, 34 or 40 or has at least 95% identity to a sequence as shown in any one of SEQ ID NO.22, 28, 34 or 40; an LCDR2 as shown in an amino acid sequence, which is shown as any one of SEQ ID NO.23, 29, 35 or 41 or has at least 95% identity to a sequence as shown in any one of SEQ ID NO.23, 29, 35 or 41; and an LCDR3 as shown in an amino acid sequence, which is shown as any one of SEQ ID NO.24, 30, 36 or 42 or has at least 95% identity to a sequence as shown in any one of SEQ ID NO.24, 30, 36 or 42.

An important advantage of the anti-PD-L1 antibody or the antigen-binding fragment thereof lies in that the anti-PD-L1 antibody or the antigen-binding fragment thereof has a high affinity and high specificity for binding to PD-L1.

An important advantage of the anti-PD-L1 antibody or the antigen-binding fragment thereof lies in that the anti-PD-L1 antibody or the antigen-binding fragment thereof has the capability of blocking the interaction between the PD-L1 expressed on a cell surface and PD-1.

An important advantage of the anti-PD-L1 antibody or the antigen-binding fragment thereof lies in that the anti-PD-L1 antibody or the antigen-binding fragment thereof can stimulate the production of cytokine.

Therefore, the anti-PD-L1 antibody or the antigen-binding fragment thereof may be used as or used for preparing antibody drugs.

In the application, a Kabat numbering system is used to label the CDR, but other methods of labeling the CDR also fall within the scope of protection of the application.

In the application, a Kabat numbering system is used to label the CDR, but other methods of labeling the CDR also fall within the scope of protection of the application.

In the present invention, as used herein, the term of "antibody or antigen-binding fragment thereof" is a protein binding a specific antigen, which refers broadly to all proteins and protein fragments including a CDR. The term "antibody" refers specifically to full-length antibodies, and the term "full-length antibody" includes both polyclonal antibodies and monoclonal antibodies. The term "antigen-binding fragment" is a substance containing a part or all of antibody CDRs, and the fragment lacks at least some of amino acids present in a full-length chain but can still be capable of specifically binding to an antigen. Such fragments are biologically-active because the fragments bind to target antigens and may competitively bind to given epitope with other antigen-binding molecules (including a complete antibody).

As used herein, the term "CDR" refers to hypervariable regions of heavy and light chains of immunoglobulins. There are three heavy chain CDRs and three light chain CDRs. Herein, depending on the situation, the term "CDR" and "CDRs" are used for referring to regions containing one or more or even all of major amino acid residues that contribute to the binding affinity of an antibody or an antigen-binding fragment thereof to the antigen or epitope by which the antibody or the antigen-binding fragment thereof recognizes.

As used herein, the term "specific binding" or similar expressions refer to the binding of an antibody or an antigen-binding fragment thereof to predetermined epitope on an antigen. Generally, the antibody or the antigen-binding fragment thereof binds with an affinity (KD) of about less than 10⁻⁶M, for example, about less than 10⁻⁷M, 10⁻⁸M, 10⁻⁹M, or 10⁻¹⁰M or less. KD is the ratio of a dissociation rate to a binding rate (koff/kon), and can be measured by methods familiar to those skilled in the art.

In some implementations, the antibody includes a heavy chain variable region and a light chain variable region. An amino acid sequence of the heavy chain variable region is shown in any one of SEQ ID NO: 5, 9, 13 or 17 or has at least 95% identity to a sequence as shown in any one of SEQ ID NO: 5, 9, 13 or 17.

In some implementations, an amino acid sequence of the light chain variable region is shown in any one of SEQ ID NO: 6, 10, 14 or 18 or has at least 95% identity to a sequence as shown in any one of SEQ ID NO: 6, 10, 14 or 18.

In the present invention, the antibody has a constant region. A heavy chain constant region is selected from any one of IgG1, IgG2, IgG3, IgG4, IgA, IgD, IgE or IgM; and a light chain constant region is a κ chain or a λ chain.

In some implementations, the species in the constant region is derived from human or mice.

In some implementations, the antibody is any one or several of a bispecific antibody, a CDR grafted antibody or a polymer antibody.

In some implementations, the antigen-binding fragment is any one or several of scFv, Fab, Fab', F (ab')2, Fv, and a single domain antibody.

In some implementations, the PD-L1 is human, monkey or mouse PD-L1.

Some implementations of the application further disclose a nucleic acid. The nucleic acid includes a first nucleic acid encoding a heavy chain variable region of the antibody or the antigen-binding fragment thereof, and/or a second nucleic acid encoding a light chain variable region of the antibody or the antigen-binding fragment thereof.

The nucleic acid is usually RNA or DNA, and the nucleic acid molecule may be single-stranded or double-stranded, but in some implementations, the nucleic acid is double-stranded DNA. The nucleic acid is "effectively linked" when being placed in a functional relationship with another nucleic acid sequence. For example, if a promoter or an enhancer affects the transcription of an encoding sequence, the promoter or the enhancer is effectively linked to the encoding sequence. In some implementations, the nucleic acid is the DNA when being linked into a vector. In addition, in view of the antibody being a membrane protein, the nucleic acid usually carries a signal peptide sequence.

In some implementations, a base sequence of the first nucleic acid is shown in any one of SEQ ID NO: 3, SEQ ID NO: 7, SEQ ID NO: 11 or SEQ ID NO: 15 or has at least 95% identity to a sequence as shown in any one of SEQ ID NO: 3, SEQ ID NO: 7, SEQ ID NO: 11 or SEQ ID NO: 15.

In some implementations, a base sequence of the second nucleic acid is shown in any one of SEQ ID NO: 4, SEQ ID NO: 8, SEQ ID NO: 12 or SEQ ID NO: 16 or has at least 95% identity to a sequence as shown in any one of SEQ ID NO: 4, SEQ ID NO: 8, SEQ ID NO: 12 or SEQ ID NO: 16.

Some implementations of the application further disclose a vector. The vector includes the nucleic acid.

As used herein, the term "vector" refers to a nucleic acid delivery vehicle into which polynucleotide may be inserted. When the vector enables the expression of a protein encoded by the inserted polynucleotide, the vector is called an expression vector. The vector may be introduced into a host cell by means of transformation, transduction or transfection, so as to obtain the expression of a genetic material elements carried by the vector in the host cell. The vector is well known to those of skill in the art, and includes, but is not limited to: a plasmid; a phagemid; a cosmid; an artificial chromosome, such as a Yeast Artificial Chromosome (YAC), a Bacteriophage Artificial Chromosome (BAC) or a P1-derived Artificial chromosome (PAC); and a phage, such as a λ phage or an M13 phage and animal viruses. The animal viruses that may be used as the vectors include, but are not limited to, retroviruses (including lentiviruses), adenoviruses, adeno-associated viruses, herpesviruses (such as herpes simplex virus), poxviruses, baculoviruses, papillomaviruses, and papovavirus (such as SV40).

Some implementations of the application further disclose a cell. The cell includes the nucleic acid or the vector.

In some implementations, the cell is an eukaryotic mammalian cell.

In an optional implementation, the cell is a Chinese hamster CHO cell.

Some implementations of the application further disclose a pharmaceutical composition. The pharmaceutical composition includes the anti-PD-L1 antibody or the antigen-binding fragment thereof, the nucleic acid, the vector or the cell.

In some implementations, the pharmaceutical composition further includes a pharmaceutically acceptable carrier.

As used herein, the term "pharmaceutical composition" exists in a form that allows the biological activity of active ingredients to be effective, and does not include additional ingredients that are unacceptably toxic to a subject which is about to apply the composition.

As used herein, the term "pharmaceutically acceptable carrier" may include any and all physiologically compatible solvents, dispersion media, coatings, antibacterial agents and antifungal agents, isotonic agents, and delayed absorption agents, which are used to prolong the shelf life or potency of antibodies.

In addition, the applications of the anti-PD-L1 antibody or the antigen-binding fragment thereof, and the nucleic acid, the vector, the cell or the pharmaceutical composition related to the anti-PD-L1 antibody or the antigen-binding fragment thereof in preparation of drugs for treating PD-L1-mediated diseases or symptoms should also be within the scope of protection of the application.

The application has the following beneficial effects.

The application provides the anti-PD-L1 antibody or the antigen-binding fragment thereof, and the anti-human PD-L1 antibody with the high affinity and high specificity for binding to the PD-L1 protein. The antibody can block the interaction between PD-L1 expressed on the cell surface and the PD-1, and shows the biological function activity of stimulating the production of the cytokine in an in vitro cell function experiment. Therefore, the anti-PD-L1 antibody or the antigen-binding fragment thereof, the nucleic acid, the vector, the cell or the pharmaceutical composition of the application has wide application prospects in preparation of drugs for treating PD-L1-mediated diseases or symptoms.

### Embodiments

### Embodiment 1

### Production of Anti-human PD-L1 Antibody

### 1. Immunogen

A recombinant human PD-L1 fusion protein was used as an immunogen, and was used for producing a human PD-L1 antibody. During immunization, a human PD-L1 extracellular domain fusion protein hPD-L1-mFc (Sino biological) carrying an mFc label was used as the immunogen.

### 2. Immunization process

A commonly used mouse hybridoma system was used to produce an anti-human PD-L1 mouse monoclonal antibody. A process included the following.

0.25mg/mL of a recombinant human PD-L1 recombinant protein and a Freund's complete adjuvant (Sigma) were mixed in equal volume, so as to obtain an oily emulsion, and the oily emulsion was subcutaneously applied to 6-week-old female BALB/c mice (females at the Guangdong Medical Experimental Animal Center) at a dose of 0.2mL. 7 days after the first immunization, the recombinant human PD-L1 recombinant protein and a Freund's incomplete adjuvant (Sigma) were mixed in equal volume, and then peritoneal immunization was performed; after immunization to 4-5 injections, tail blood was collected for potency testing; after potency reached to established titer, 0.1mL of the immunogen diluted by normal saline was used for immunological facilitation; and splenic fusion was performed 3 days later.

### 3. Preparation of hybridoma cells

Mouse tumor cells and immunized spleen cells were mixed in a ratio of 1:2 cell numbers, were transferred into a 50 mL centrifuge tube, and were washed once with an RPMI1640 basic culture medium. Supernatant was discarded; the cells were well mixed with 18 mL of an electrofusion solution (BTX), and were added into an electrofusion groove; and electrofusion was performed according to established parameters. After fusion, 100 mL of the RPMI1640 basic culture medium was added to perform gentle suspending, and was evenly divided in 96-well plates, 20 pieces in total; and culture was performed in a 37°C and 5%CO₂ cell incubator in a standing manner in 50µL/well. Culture was performed to Day 6, an HT-containing RPMI1640 complete culture medium was replaced once.

### 4. Detection of anti-human PD-L1 clone supernatant

A 0.05M carbonate buffer solution was used to dilute a home-made human PD-L1 recombinant protein carrying a human Fc label to a final concentration of 1µg/mL, and was added into a 96-well ELISA detection plate in 100 µL/well; and coating was performed overnight at 2°C-8°C. Supernatant was discarded; a blocking solution (1×PBS+1%BSA) was added in 200µL/well; and blocking was performed for 1h at 37°C. At Day 7 after fusion, cell supernatant was added in 100 µL/well, and incubation was performed for 30 min at 37°C in a standing manner. Washing was performed for 3 times with 1×PBS. HRP-labeled goat anti-mouse IgG (sigma) was added in 100 µL/well; incubation was performed for 30 min at 37°C in a standing manner; and after washing was performed for 3 times with the 1×PBS, a color reaction was performed.

### 5. Screening of subclones

Fusions with OD450≥0.5 were selected; and cloning was performed with a limiting dilution method for at least 2 times. Detection was performed with the experimental process of S4 ; and the obtained positive subclone was cultured in vitro for conservation and expression.

### 6. Expression of anti-human PD-L1 antibody

After a clone producing the anti-human PD-L1 antibody grew until a convergence rate reached 80%-90%, cells were blown down; after centrifugation was performed for 5 min at 1000rpm, the cells were resuspended with 30 mLof an SFM complete culture medium containing 5% FBS, and the culture medium was transferred to a 150mL SF shake flask; and culture was performed for 2-3 days at 37°C, 8%CO₂ and 120rpm. When cell density reached 5×10E5/mL, centrifugation was performed for 5 min at 1000rpm; the cells were resuspended with a 50 mL serum free SFM basic culture medium, and the culture medium was transferred to a 250mL SF shake flask; culture was performed for 4-5 days at 37°C, 8%CO₂ and 120rpm; and centrifugation was performed for 20 min at 9000rpm, and supernatant is collected for clone binding identification.

### 7. Binding activity identification of fermentation supernatant

In order to evaluate the binding activity of anti-human PD-L1 clone fermentation supernatant to human PD-L1 and macaque PD-L1, Chinese hamster CHO stable cell strains stably expressing human PD-L1 full-length protein (Uniprot No.NP_0548621.1, SEQ ID NO.1) and the macaque PD-L1 (Uniprot No.XP_005581836, SEQ ID NO.2) were screened and constructed, so as to evaluate the binding activity of the anti-human PD-L1 clone fermentation supernatant. An experimental process included the following.

A 1×FCM buffer (1×PBS+3%BSA) was used to resuspend the CHO stable cell strains to 2×10E6/mL; the mixture was added into a 96-well V base plate in 100µL/well; after 250×g was taken to perform centrifugation for 5 min, supernatant was discarded in a sucking manner; the anti-human PD-L1 clone fermentation supernatant was added in 100µL/well; and incubation was performed on ice for 30 min in a standing manner, then 250×g was taken to perform centrifugation for 5 min, and the supernatant was discarded in a sucking manner. After washing was performed for 2 times with the 1×FCM buffer, iFluor^{™}633GAM fluorescent secondary antibodies diluted by the 1×FCM buffer (dilution ratio being 1:400) were added in 100µL/well (ATT bioquest); and incubation was performed on ice for 30 min. After 250×g was taken to perform centrifugation for 5 min, supernatant was discarded in a sucking manner. After washing was performed for 2 times with the 1×FCM buffer, the cells were resuspended by adding the 1×PBS in 100µL/well; and the flow cytometer (Beckman, CytoFLEX) was used for detection.

### 8. Purification

Positive clone supernatant was selected for purification. ProA affinity chromatography was used for purification. A process included the following.

An AKTA avant 150 chromatography device was used; at least a 5CV equilibration buffer (10mM PBS) was used to equilibrate a chromatography column (for example, MabSelectSuRe LX, GE); and a sample was loaded to the chromatography column, so as to cause a target protein to adsorb on the chromatography column and cause other impurities to penetrate and separate. After sample loading, the at least 5CV equilibration buffer (10mM PBS) was used to wash the chromatography column again; then an elution buffer (20mM NaAc, pH=3.4) was used to elute the target protein; a neutralizing buffer (1M Tris, pH8.0) was added in a collection tube in advance; and the adding volume of the neutralizing buffer was determined according to estimated content of an elution sample, and 10% elution volume was added generally.

### 9. Measurement of concentration of anti-human PD-L1 antibody

Concentration measurement was performed on the sample with Biotek-Epoch-Take-3; an A280 method was used to detect the concentration of the antibody, that is, with extinction coefficient E.C.=1.37, optical path=0.05mm (Take-3 plates having slight differences in the optical path of different wells, which would be automatically corrected); a light absorption value of the sample was detected by means of a device; and the concentration of an antibody to be measured was calculated according to Lambert-Beer law. If the concentration of the sample was too low, ultrafiltration concentration needed to be performed; the sample was concentrated to > 0.5 mg/mL with an ultrafiltration concentration tube (Ultra-15Centrifugal Filter Devices, 30kD) according to a general operation method provided in the specification; and a concentrated end sample was collected, and filtration sterilization were performed with a 0.22µm sterile needle filter (Cobetter, PES, 0.22µm, and diameter being 13 mm).

### 10. Identification of subtype of anti-human PD-L1 antibody

A 0.05M pH 9.5 carbonate buffer solution was used to dilute a recombinant human PD-L1 recombinant protein carrying a human Fc label to a final concentration of 1µg/mL. The mixture was added into a 96-well ELISA detection plate in 100 µL/well; and coating was performed overnight at 2°C-8°C. Supernatant was discarded in a sucking manner; a blocking solution (1×PBS+1%BSA) was added in 200µL/well; and blocking was performed for 1h at 37°C. The anti-human PD-L1 antibody was diluted to 1µg/mL with 1×PBS containing 1%BSA; the antibody was added in 100 µL/well; and incubation was performed for 30 min at 37°C. Washing was performed for 3 times with the 1×PBS. HRP-labeled goat anti-mouse IgG (sigma A0168-1ML), HRP-labeled goat anti-mouse IgG2a (thermo fisher M32207), HRP-labeled goat anti-mouse IgG1 (thermo fisher PA1-74421), HRP-labeled goat anti-mouse IgG2b (thermo fisher M32407), HRP-labeled goat anti-mouse IgG3 (thermo fisher M32607) and HRP-labeled goat anti-mouse IgM (thermo fisher 31440) were added in 100 µL/well; incubation was performed for 30 min at 37°C in a standing manner; and after washing was performed for 3 times with the 1×PBS, the color reaction was performed. The 4 anti-human PD-L1 antibodies (F016-21, F016-193, F016-568 and F016-870) were obtained.

### 11. Identification of sequence of anti-human PD-L1 antibody

Sequence retrieval and sequencing of mouse hybridoma clones of the 4 anti-human PD-L1 antibodies (F016-21, F016-193, F016-568 and F016-870) were performed by conventional methods, and details were described below.

An RNA extraction kit MagExtractor-RNA- (Toyobo) was used to obtain total RNA from 5×10⁶ mouse hybridoma cells by means of extraction. By means of a 5'RACE method process, an SMARTer RACE 5'Kit kit (Takara Bio) was used to obtain a cDNA sequence. A 5'RACE universal primer and a 3' mouse specific conserved region primer were used to extend an antibody variable region sequence, a PCR clone sequence including the variable region sequence was linked to a pMD18-T vector (Takara, Cat.D101A), and the vetor was transfected into *Escherichia coli* DH5α competent cells (NEB). An SanPrep column plasmid mini-preps kit (Sangon Biotech) was used to extract plasmid, and then sequencing was performed.

Nucleotide sequences of the variable regions of the 4 anti-human PD-L1 antibodies were shown in Table 1, and amino acid sequences were shown in Table 2.

**Table 1 Nucleotide sequences of the 4 anti-human PD-L1 antibodies**

| Anti-human PD-L1 antibody | Variable region of heavy chain | Variable region of light chain |
|---|---|---|
| F016-21 | SEQ ID NO.3 | SEQ ID NO.4 |
| F016-193 | SEQ ID NO.7 | SEQ ID NO.8 |
| F016-568 | SEQ ID NO.11 | SEQ ID NO.12 |
| F016-870 | SEQ ID NO.15 | SEQ ID NO.16 |

**Table 2 Amino acid sequences of the 4 anti-human PD-L1 antibodies**

| Anti-human PD-L1 antibody | Variable region of heavy chain | Variable region of light chain |
|---|---|---|
| F016-21 | SEQ ID NO.5 | SEQ ID NO.6 |
| F016-193 | SEQ ID NO.9 | SEQ ID NO.10 |
| F016-568 | SEQ ID NO.13 | SEQ ID NO.14 |
| F016-870 | SEQ ID NO.17 | SEQ ID NO.18 |

### Embodiment 2

### Binding Activity of Anti-human PD-L1 antibody

### 1. Detection on the binding activity of an anti-human PD-L1 antibody to a CHO empty cell (CHO-FP1).

### (1) Experimental method

Processes of detecting the binding activity of the anti-human PD-L1 antibody to the CHO-FP1 included the following.

A 1×FCM buffer (1×PBS+3%BSA) was used to resuspend the CHO-FP1 to 2×10E6/mL; and the mixture was added into a 96-well V base plate in 100µL/well. The 1×FCM buffer was used to dilute the anti-human PD-L1 antibody until a concentration reached 20µg/mL. The mixture was added into the cells in 100µL/well; and incubation was performed on ice for 30 min in a standing manner, then 250×g was taken to perform centrifugation for 5 min, and supernatant was discarded in a sucking manner. After washing was performed for 2 times with the 1×FCM buffer, iFluor^{™}633GAM fluorescent secondary antibodies (ATT bioquest) diluted by the 1×FCM buffer (dilution ratio being 1:400) were added in 100µL/well; and incubation was performed on ice for 30 min. After 250×g was taken to perform centrifugation for 5 min, supernatant was discarded in a sucking manner. After washing was performed for 2 times with the 1×FCM buffer, the cells were resuspended by adding 1×PBS in 100µL/well; and the flow cytometer (Beckman, CytoFLEX) was used for detection. Avelumab-mlgG2a (mlgG2a subtype), atezolizumab-mlgG1 (mlgG1 subtype), mlgG1 homotype, mlgG2a homotype and APC anti-mlgG were used as control antibodies.

### (2) Experimental result

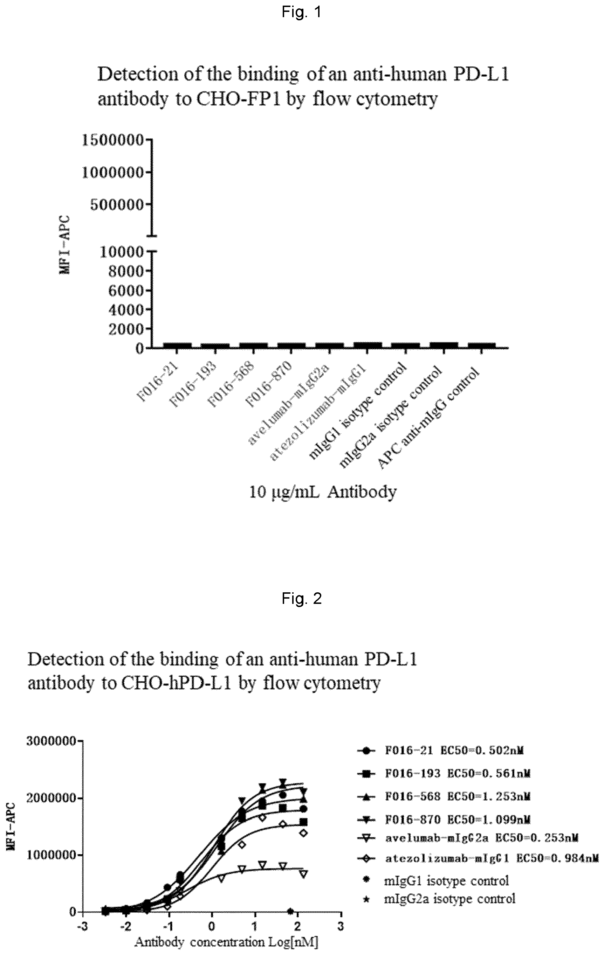

Detection results of the binding activity of the anti-human PD-L1 antibody to the CHO-FP1 were shown in Fig. 1. The results showed that the 4 anti-human PD-L1 antibodies did not bind to the CHO-FP1.

### 2. Detection on the binding activity of the anti-human PD-L1 antibody to CHO-hPD-L1 stably expressing human PD-L1.

### (1) Experimental method

In order to represent the binding affinity of the anti-human PD-L1 antibody to a membrane protein PD-L1, the 1×FCM buffer (1×PBS+3%BSA) was used to dilute the anti-human PD-L1 antibody in 3x multiple proportions, and an initial concentration was 60µg/mL; and then co-incubation was performed with a CHO-hPD-L1 stable cell strain. A specific experimental process was the same as S1. Avelumab-mlgG2a (mlgG2a subtype), atezolizumab-mlgG1 (mlgG1 subtype), mlgG1 homotype and mlgG2a homotype were used as control antibodies.

### (2) Experimental result

Detection results of the binding activity of the anti-human PD-L1 antibody to the CHO-hPD-L1 stably expressing human PD-L1 were shown in Fig. 2.

Results showed that, the binding activity EC50 of the 4 anti-human PD-L1 antibodies to the CHO-hPD-L1 was 0.5-1.3nM, and the binding activity EC50 of the control antibodies to the CHO-hPD-L1 was 0.2-1.0nM.

### 3. Detection on the binding activity of an anti-human PD-L1 antibody to CHO-cynoPD-L1 stably expressing macaque PD-L1.

### (1) Experimental method

In order to represent the binding affinity of the anti-human PD-L1 antibody to macaque PD-L1, the 1×FCM buffer (1×PBS+3%BSA) was used to dilute the anti-human PD-L1 antibody in 3x multiple proportions, and an initial concentration was 30µg/mL; and then co-incubation was performed with a CHO-cynoPD-L1 stable cell strain. A specific experimental process was the same as S1. Avelumab-mlgG2a (mlgG2a subtype), atezolizumab-mlgG1 (mlgG1 subtype), mlgG1 homotype and mlgG2a homotype were used as control antibodies.

### (2) Experimental result

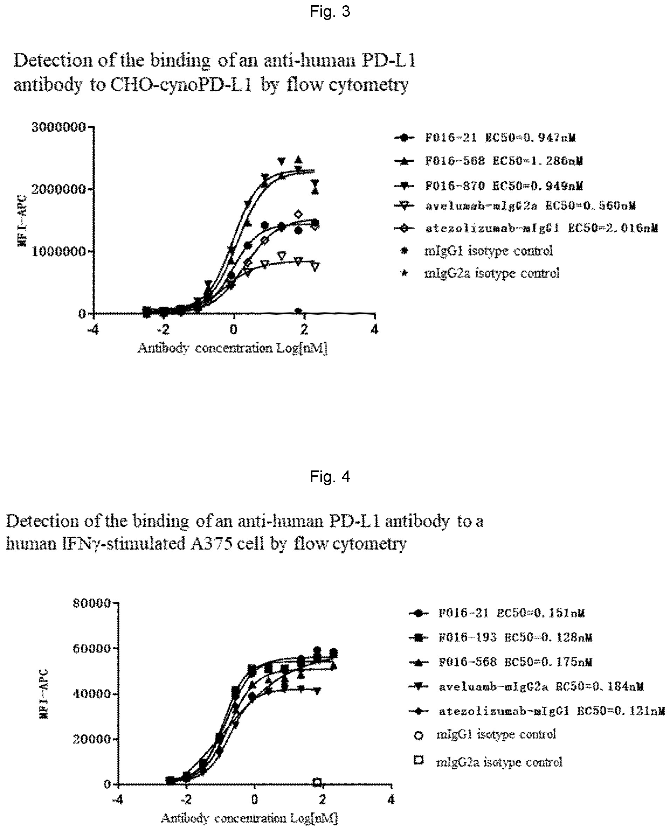

Detection results of the binding activity of the anti-human PD-L1 antibody to the CHO-cynoPD-L1 stably expressing macaque PD-L1 were shown in Fig. 3. Results showed that, the binding activity EC50 of the 4 anti-human PD-L1 antibodies to the CHO-cynoPD-L1 was 0.9-1.3nM, and the binding activity EC50 of the control antibodies to the CHO-cynoPD-L1 was 0.5-2.1nM.

### 4. Detection on the binding activity of an anti-human PD-L1 antibody to an IFNγ-stimulated A375 cell.

### (1) Experimental method

The binding activity of the anti-human PD-L1 antibody to the PD-L1 membrane protein was detected with human IFNγ to stimulate the expression of PD-L1 on a tumor cell surface. An experimental process included the following.

50ng/mL of the human IFNγ (R&D) was used to stimulate the expression of human PD-L1 on human melanoma cells A375 (Shanghai cell bank) overnight; on the next day, 0.25% pancreatin (Gibco) was used to digest the A375 cells; after washing was performed once with DPBS, live cell density was adjusted to 2E6/mL; and the mixture was added into the 96-well V base plate in 100µL/well. The 1×FCM buffer was used to dilute the anti-human PD-L1 antibody until a concentration reached 60µg/mL. The mixture was added into the cells in 100µL/well; and incubation was performed on ice for 30 min in a standing manner, then 250×g was taken to perform centrifugation for 5 min, and supernatant was discarded in a sucking manner. After washing was performed for 2 times with the 1×FCM buffer, iFluor^{™}633GAM fluorescent secondary antibodies (ATT bioquest) diluted by the 1×FCM buffer (dilution ratio being 1:400) were added in 100µL/well; and incubation was performed on ice for 30 min. After 250×g was taken to perform centrifugation for 5 min, supernatant was discarded in a sucking manner. After washing was performed for 2 times with the 1×FCM buffer, the cells were resuspended by adding the 1×PBS in 100µL/well; and the flow cytometer (Beckman, CytoFLEX) was used for detection. Avelumab-mlgG2a (mlgG2a subtype), atezolizumab-mlgG1 (mlgG1 subtype), mlgG1 homotype and mlgG2a homotype were used as control antibodies.

### (2) Experimental result

Detection results of the binding activity of the anti-human PD-L1 antibody to the IFNγ-stimulated A375 cell were shown in Fig. 4. Results showed that, the binding activity EC50 of the anti-human PD-L1 antibody to the IFNγ-stimulated A375 cell was 0.12-0.18nM, and the binding activity EC50 of the control antibodies to the IFNγ-stimulated A375 cell was 0.12-0.19nM.

### 5. Analysis of binding kinetics KD of anti-human PD-L1 antibody to human PD-L1

### (1) Experimental method

Binding kinetic constant analysis of the anti-human PD-L1 antibody and human PD-L1 was performed by an MD ForteBIOQK^{e} platform. An experimental method included the following.

An equilibration buffer (1×PBS+0.02%Tween20) was used to dilute a home-made human PD-L1 extracellular domain recombinant protein hPD-L1-his carrying a his label, until a final concentration reached 5µg/mL. The equilibration buffer (1×PBS+0.02%Tween20) was used to dilute the 4 anti-human PD-L1 antibodies in 2x multiple proportions, an initial concentration was 100nM, and there were 7 concentrations in total. After an anti-Penta-HIS biosensor (ForteBIO) was fully hydrated, the hPD-L1-his recombinant protein was first cured for 150 seconds, equilibration was performed for 90 seconds, and then the recombinant protein was bound to the anti-human PD-L1 antibody, binding time was 180 seconds, and dissociation time was 600 seconds. The whole reaction was undergone at 25°C and 1000rpm. Finally, curve fitting was performed with Octet analysis software, so as to obtain a binding kinetic constant KD of the anti-human PD-L1 antibody. Avelumab-mlgG2a (mlgG2a subtype) and atezolizumab-mlgG1 (mlgG1 subtype) were used as control antibodies.

### (2) Experimental result

Analysis results of the binding kinetics KD of the anti-human PD-L1 antibody to the human PD-L1 were shown in Table 3. Results showed that, as same as the control antibodies, the binding kinetic constants of the 4 anti-human PD-L1 antibodies to the human PD-L1 recombinant protein were all at a pM level.

**Table 3 Analysis result of binding kinetics KD of anti-human PD-L1 antibody to human PD-L1**

| Antibody name | KD, × 10E⁻¹⁰ (M) |
|---|---|
| F016-21 | 0.566 |
| F016-193 | 0.098 |
| F016-568 | 1.010 |
| F016-870 | 0.742 |
| avelumab-mlgG2a | 0.943 |
| atezolizumab-mlgG1 | 0.565 |

### Embodiment 3

### Epitope analysis of Anti-human PD-L1 Antibody

### 1. Epitope binning

### (1) Experimental method

Epitope competition analysis was performed on the anti-human PD-L1 antibody with an MD ForteBIOQKe platform. An experimental method included the following.

An equilibration buffer (1×PBS+0.02%Tween20) was used to dilute a home-made human PD-L1 extracellular domain recombinant protein hPD-L1-his carrying a his label, until a final concentration reached 5µg/mL. The equilibration buffer (1×PBS+0.02%Tween 20) was used to dilute the anti-human PD-L1 antibody, and the final concentration was 20 µg/mL. After an anti-Penta-HIS biosensor (ForteBIO, Cat.18-5122) was fully hydrated, the hPD-L1-his recombinant protein was first cured for 150 seconds, equilibration was performed for 90 seconds, and the recombinant protein bound to the first anti-human PD-L1 antibody for 120 seconds; equilibration was performed for 90 seconds, and then the recombinant protein bound to the second anti-human PD-L1 antibody, and binding time was 120 seconds. The whole reaction was undergone at 25°C and 1000rpm. Finally, Octet analysis software was used to perform epitope binning analysis.

### (2) Experimental result

Epitope binning analysis results showed that F016-21 and F016-193 did not compete with avelumab.

### 2. Analysis of antibody epitope competition at cellular level

### (1) Experimental method

The 1×FCM buffer (1×FBS+3%BSA) was used to dilute the anti-PD-L1 antibody; the first antibody included avelumab-hlgG1 (hlgG1 subtype), and a hlgG1 isotype control antibody, with a concentration being 40µg/mL; and the second antibody included the anti-human PD-L1 antibody, avelumab-mlgG2a (mlgG2a subtype), and a mlgG2a isotype control antibody, with a concentration being 2µg/mL. The 1×FCM buffer was used to resuspend the CHO-hPD-L1 stable cell strain; cell density was adjusted to be 2×10E6/mL; the cells were spread in the 96-well plate in 100µL/well; 50µL of the first antibody was added; incubation was performed on ice for 30 min; and then 50µL of the second antibody was added, and incubation was performed on ice for 30 min. After 250×g was taken to perform centrifugation for 5 min, supernatant was discarded in a sucking manner. After washing was performed for 2 times with the 1×FCM buffer, iFluor^{™}633GAM fluorescent secondary antibodies (ATT bioquest) diluted by the 1×FCM buffer (dilution ratio being 1:400) were added in 100µL/well; and incubation was performed on ice for 30 min. After 250×g was taken to perform centrifugation for 5 min, supernatant was discarded in a sucking manner. After washing was performed for 2 times with the 1×FCM buffer, the cells were resuspended by adding the 1×PBS in 100µL/well; and the flow cytometer (Beckman, CytoFLE×) was used for detection.

### (2) Experimental result

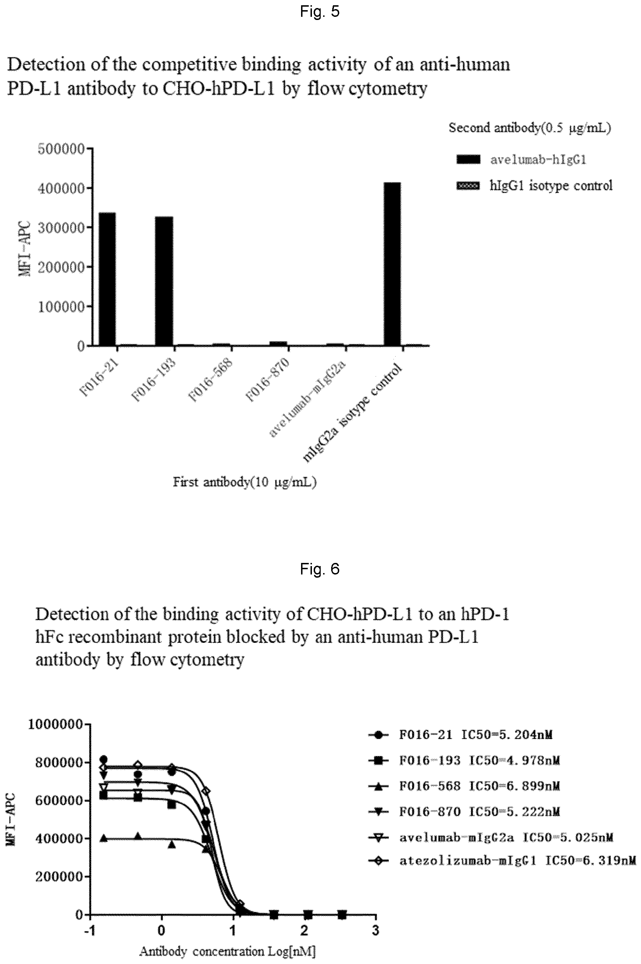

Detection results of the competitive binding activity of the anti-human PD-L1 antibody to the CHO-hPD-L1 were shown in Fig. 5. F016-21 and F016-193 did not compete with avelumab.

### Embodiment 4

### Anti-human PD-L1 Antibody Mediated Ligand Blocking Experiment.

1. Binding and blocking activity with PD-1.

The PD-L1 protein expressed by tumor cells or antigen-presenting cells bound to the PD-L1 protein expressed by a lymphocyte surface, so as to inhibit the stimulation activity of lymphocyte. The blocking anti-human PD-L1 antibody binding PD-1 and PD-L1 was evaluated, and evaluation may be performed by means of experimental methods 1 and 2. The experimental methods 1 and 2 were respectively as follows.

### (1) Experimental method 1

### (1) Experimental method

The 1×FCM buffer was used to dilute the anti-human PD-L1 antibody in 3x multiple proportions, and an initial concentration was 200µg/mL. The 1×FCM buffer was used to dilute the self-produced recombinant human PD-L1 protein carrying hFc, until the concentration reached 4µg/mL. The 1×FCM buffer was used to resuspend the CHO-hPD-L1 cells to the cell density of 2×10E6/mL, and the cells were evenly spread in the 96-well V base plate in 100µL/well. The antibodies were added into the CHO-hPD-L1 cells in 50 µL/well; incubation was performed on ice for 30 min; recombinant human PD-1-hFc protein was added in 50 µL/well; and incubation was performed on ice for 30 min After 250×g was taken to perform centrifugation for 5 min, supernatant was discarded in a sucking manner; after washing was performed once with the 1×FCM buffer, APC-labeled goat anti-mouse Fc fluorescent secondary antibodies (Biolegend) diluted by the 1×FCM buffer (dilution ratio being 1:500) were added in 100µL/well; and incubation was performed on ice for 30 min. After 250×g was taken to perform centrifugation for 5 min, supernatant was discarded in a sucking manner. After washing was performed for 2 times with the 1×FCM buffer, the cells were resuspended by adding the 1×PBS in 100µL/well; and the flow cytometer (Beckman, CytoFLEX) was used for detection. Avelumab-mlgG2a (mlgG2a subtype) and atezolizumab-mlgG1 (mlgG1 subtype) were used as control antibodies.

### (2) Experimental result

Detection results of the binding activity of the CHO-hPD-L1 to the hPD-L1 hFc recombinant protein blocked by the anti-human PD-L1 antibody in the experimental method 1 were shown in Fig. 6. Results showed that, the binding activity IC50 of PD-1 to PD-L1 blocked by 4 mouse monoclonal antibodies was 4.9-6.9nM, and the binding activity IC50 of the PD-1 to the PD-L1 blocked by the control antibodies was 5.0-6.4nM.

### (2) Experimental method 2

### (1) Experimental method

The 1×FCM buffer was used to dilute the anti-human PD-L1 antibody in 3x multiple proportions, and an initial concentration was 200µg/mL. The 1×FCM buffer was used to dilute the self-produced recombinant human PD-L1 protein carrying hFc, until the concentration reached 2µg/mL. The 1×FCM buffer was used to resuspend the CHO-hPD-1 cells to the cell density of 2×10E6/mL, and the cells were evenly spread in the 96-well V base plate in 100µL/well. The antibodies were added into the CHO-hPD-1 cells in 50 µL/well; incubation was performed on ice for 30 min; recombinant human PD-L1-hFc protein was added in 50 µL/well; and incubation was performed on ice for 30 min. After 250×g was taken to perform centrifugation for 5 min, supernatant was discarded in a sucking manner; after washing was performed once with the 1×FCM buffer, APC-labeled goat anti-mouse Fc fluorescent secondary antibodies (Biolegend) diluted by the 1×FCM buffer (dilution ratio being 1:500) were added in 100µL/well; and incubation was performed on ice for 30 min. After 250×g was taken to perform centrifugation for 5 min, supernatant was discarded in a sucking manner. After washing was performed for 2 times with the 1×FCM buffer, the cells were resuspended by adding the 1×PBS in 100µL/well; and the flow cytometer (Beckman, CytoFLEX) was used for detection.

Avelumab-mlgG2a (mlgG2a subtype) and atezolizumab-mlgG1 (mlgG1 subtype) were used as control antibodies.

### (2) Experimental result

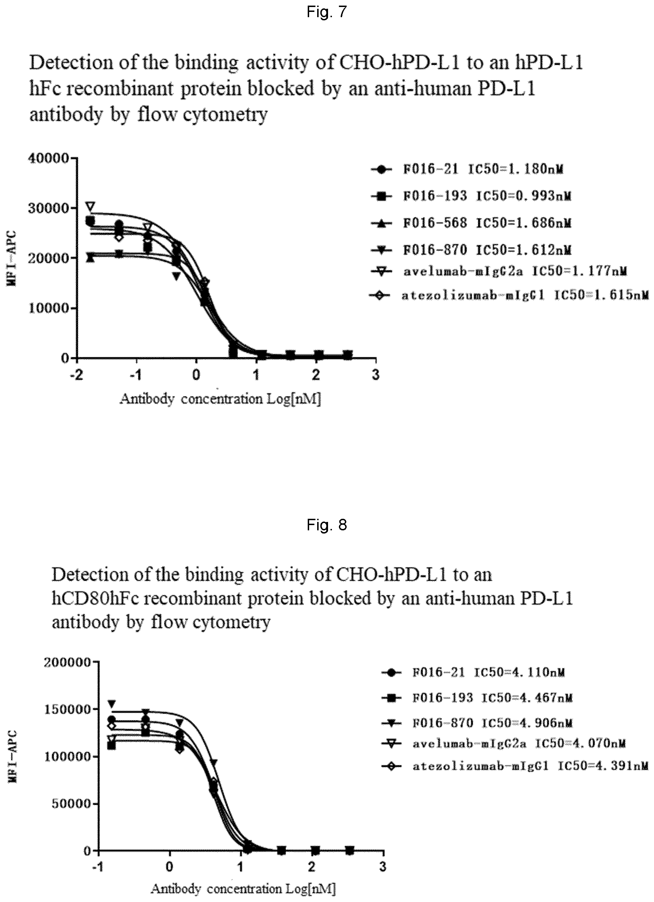

Detection results of the binding activity of the CHO-hPD-L1 to the hPD-L1 hFc recombinant protein blocked by the anti-human PD-L1 antibody in the experimental method 2 were shown in Fig. 7. Results showed that, the binding activity IC50 of PD-1 to PD-L1 blocked by 4 mouse monoclonal antibodies was 0.9-1.7nM, and the binding activity IC50 of the PD-1 to the PD-L1 blocked by the control antibodies was 1.1-1.6nM.

### 2. Blocking activity of binding to CD80.

### (1) Experimental method

The PD-L1 protein expressed by tumor cells or antigen-presenting cells bound to the CD80 protein expressed by a lymphocyte surface, so as to inhibit the stimulation activity of lymphocyte. The activity of anti-human PD-L1 antibody blocking the binding of PDL1 and CD80 was evaluated, and the experimental method was as follows.

The 1×FCM buffer was used to dilute the anti-human PD-L1 antibody in 3x multiple proportions, and an initial concentration was 200µg/mL. The 1×FCM buffer was used to dilute the self-produced recombinant human CD80 protein, until the concentration reached 160µg/mL. The 1×FCM buffer was used to resuspend the CHO-hPD-L1 cells to the cell density of 2×10E6/mL, and the cells were evenly spread in the 96-well V base plate in 100µL/well.

The antibodies were added into the CHO-hPD-L1 cells in 50 µL/well; incubation was performed on ice for 30 min; recombinant human CD80-hFc protein was added in 50 µL/well; and incubation was performed on ice for 30 min. After 250×g was taken to perform centrifugation for 5 min, supernatant was discarded in a sucking manner; after washing was performed once with the 1×FCM buffer, APC-labeled goat anti-mouse Fc fluorescent secondary antibodies (Biolegend) diluted by the 1×FCM buffer (dilution ratio being 1:500) were added in 100µL/well; and incubation was performed on ice for 30 min. After 250×g was taken to perform centrifugation for 5 min, supernatant was discarded in a sucking manner. After washing was performed for 2 times with the 1×FCM buffer, the cells were resuspended by adding the 1×PBS in 100µL/well; and the flow cytometer (Beckman, CytoFLEX) was used for detection.

Avelumab-mlgG2a (mlgG2a subtype) and atezolizumab-mlgG1 (mlgG1 subtype) were used as control antibodies.

### (2) Experimental result

The results of anti-human PD-L1 antibody to block CHO-hPDL1 and recombinant hCD80-hFc protein interaction were shown in Fig. 8. The results showed that, the IC50 of the 4 mouse monoclonal antibodies to block PD-L1 and CD80 interaction was 4.1-5.0nM, and the IC50 of the control antibodies to block PD-L1 and CD80 interaction was 4.0-4.4nM.

### Embodiment 5

### In vitro Activity Characterization of Anti-human PD-L1 antibody

### 1. Detection of luciferase reporter gene system under co-incubation of Jurkat-GL4.30-hPD-1 and CHO-hPD-L1-OKT3

### (1) Experimental method

In order to use a luciferase reporter gene system to detect the in vitro functional activity of an anti-human PD-L1 mouse monoclonal antibody, a lentivirus packaging system was used to construct a Jurkat-GL4.30-hPD-1 effector cell strain stably expressing hPD-1 and luciferase genes. At the same time, a PD-L1 presenting cell CHO-hPD-L1-OKT3 was constructed from Chinese hamster ovary (CHO) cells that stably expressing human PD-L1 and the antigen-independent TCR cell surface kinesin. On the day of the experiment, a 1%FBS-containing RPMI1640 complete culture medium was used to resuspend CHO-hPD-L1-OKT3 cells and Jurkat-GL4.30-hPD-1 cells; live cell density was respectively 1×10E6/mL and 5×10E6/mL; and the cells were spread in a 96-well fluorescent enzyme labeling plate in 40µL/well. The 1 % FBS-containing RPMI1640 complete culture medium was used to dilute the anti-human PD-L1 mouse monoclonal antibody, and an initial concentration was 50µg/mL; the antibodies were sucked into mixed cells of the Jurkat-GL4.30-hPD-1 cells and the CHO-hPD-L1-OKT3 cells in 20µL/well; after well mixing, culture was performed in a cell incubator for 6h at 37°C and 5%CO₂. A Bright-Lumi^{™} solution (Beyotime) thawed in advance was added in 100µL/well; and standing was performed for 5 min in dark, and then a multi-functional microplate reader (Molecular Device, SpectraMax i3x multi-functional microplate reader) was used for detection in a Lumi mode. Avelumab-mlgG2a (mlgG2a subtype), atezolizumab-mlgG1 (mlgG1 subtype), mlgG1 homotype and mlgG2a homotype were used as control antibodies.

### (2) Experimental result

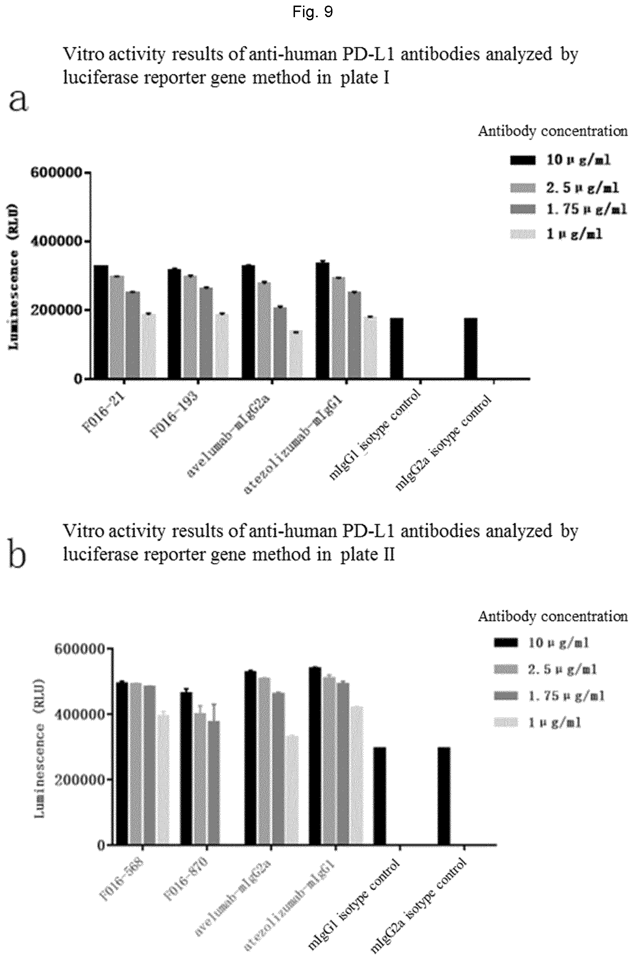

The in vitro activity results of anti-human PD-L1 antibodies analyzed by luciferase reporter gene method in plate I and plate II were respectively shown in Fig. 9. Results showed that, 4 antibodies could mediate the callback of a luciferase reporter gene signal, and showed antibody dose dependence.

### 2. Stimulation of production of IL-2 and IFN gamma in T-DC allogeneic mixed lymphatic response system

### (1) Experimental method

By means of an allogeneic T-DC MLR experiment, the activity of the anti-human PD-L1 antibody to stimulate cytokine IL-2 and IFN gamma was evaluated. An experimental process included the following.

Peripheral Blood Mononuclear Cells (PBMC) were isolated from healthy peripheral blood with a human lymphocyte isolation solution Lymphoprep^{™} (Axis-Shield). The PBMC of a donor 1 was positively screened by human CD14 Microbeads (Miltenyi) to obtain CD14⁺ mononuclear cells. The mononuclear cells were inoculated into a T75 culture flask according to 5×10E5/mL; cytokine human GM-CSF and IL-4 were replenished, until a final concentration reached 50ng/mL; after 6 days of continuous stimulation, human TNF alpha was replenished, until the final concentration reached 50ng/mL; and induced differentiation was continued for 3 days, so as to obtain mature DC cells. The PBMC of a donor 2 was negatively screened by EasySep^{™} Human T Cell Enrichment Kit (Stemcell), so as to obtain CD3⁺T cells. According to a DC:T cell ratio being 1:5 and DC cell mass being 2×10E4/well, DC and T cells were well mixed, and then spread in a 96-well U-shaped plate for a total of 150 µL/well system. An X-VIVO 15 complete culture medium was used to dilute the anti-human PD-L1 antibody, and the initial concentration was 20µg/mL; dilution was performed in 4x multiple proportions; and the antibody was added to the cells in 50µL/well. 3-5 days after the mixed lymphatic experimental response, the expression of IL-2 and IFN gamma in cell supernatant was detected. Avelumab-mlgG2a (mlgG2a subtype), mlgG1 homotype and mlgG2a homotype were used as control antibodies.

### (2) Experimental result

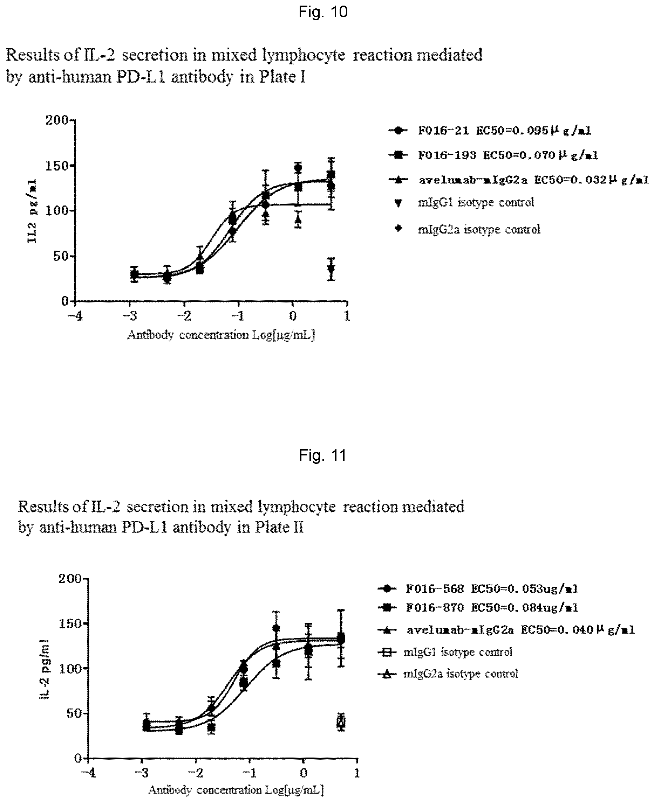

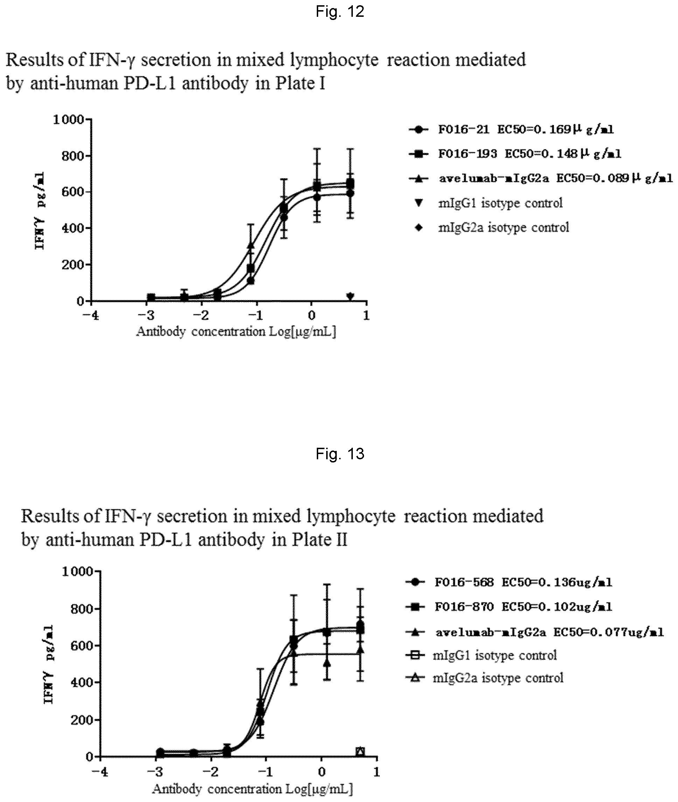

The results of IL-2 secretion in mixed lymphocyte reaction mediated by anti-human PD-L1 antibody in plate I and plate II were respectively shown in Fig. 10 and Fig. 11. The EC50 was successively 0.07-0.1µg/mL and 0.05-0.09µg/mL, and the EC50 of the control antibodies was successively 0.03µg/mL and 0.04µg/mL. The results of IFN-γ secretion in mixed lymphocyte reaction mediated by anti-human PD-L1 antibody in plate I and plate II were respectively shown in Fig. 12 and Fig. 13. The EC50 was successively 0.14-0.17µg/mL and 0.1-0.14µg/mL, and EC50 of the control antibodies was successively 0.089µg/mL and 0.077µg/mL. Results showed that, in the T-DC allogeneic mixed lymphatic response system, the anti-human PD-L1 antibody can mediate the upregulation of cytokine IL-2 and IFNγ, and was positively correlated with antibody dosage.

### Embodiment 6

### Binding Specificity Analysis of Anti-human PD-L1 Antibody

The PD-L1 protein belongs to the B7 family of proteins, and fellow family proteins of the protein included PD-L2 (B7-DC), ICOSL (B7-H2), B7-H3 CD80 (B7-1), CD86 (B7-2), etc. In order to detect the binding specificity analysis of anti-human PD-L1 antibody, an experimental process included the following.

A 50mM CB buffer was used to dilute a recombinant human PD-L1 protein carrying a hFc label, a human PD-L2 protein (Acrobiosystem), a human ICOSLG protein (Acrobiosystem), a human B7-H3 protein (Acrobiosystem), a human CD28 protein (Acrobiosystem), a human CD86 protein (Acrobiosystem) and a human CTLA-4 protein (Acrobiosystem) to 1µg/mL; and the proteins were added into a 96-well ELISA detection plate in 100 µL/well, and were coated overnight at 2°C-4°C. Supernatant was discarded; a blocking solution (1×PBS+1%BSA) was added in 200µL/well; and blocking was performed for 1h at 37°C. A 1 % BSA-containing PBS was used to dilute the anti-human PD-L1 antibody to 10µg/mL; the antibody was added in 100 µL/well; and incubation was performed for 30 min at 37°C in a standing manner. Washing was performed for 3 times with the 1×PBS. HRP-labeled goat anti-mouse IgG (sigma A0168-1ML) was added in 100 µL/well; incubation was performed for 30 min at 37°C in a standing manner; and after washing was performed for 3 times with the 1×PBS, a color reaction was performed.

### 2. Experimental result

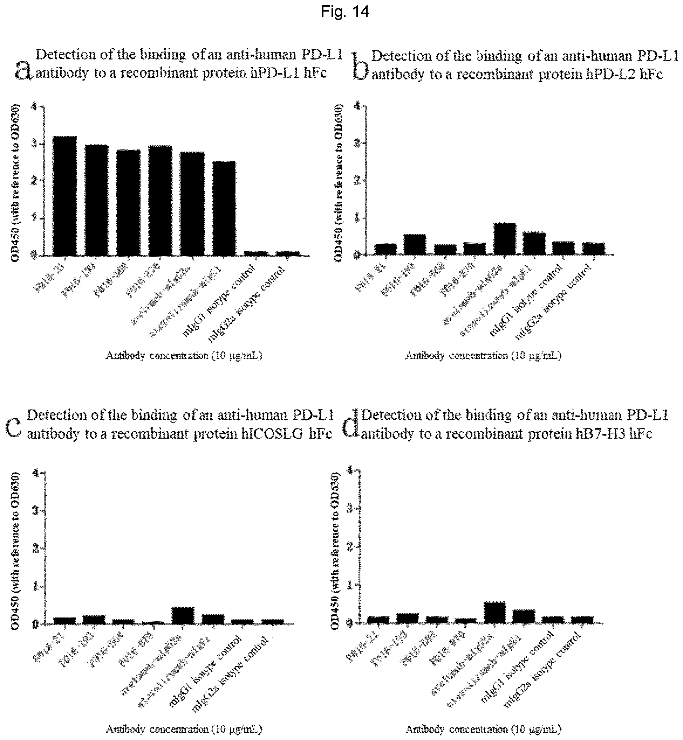

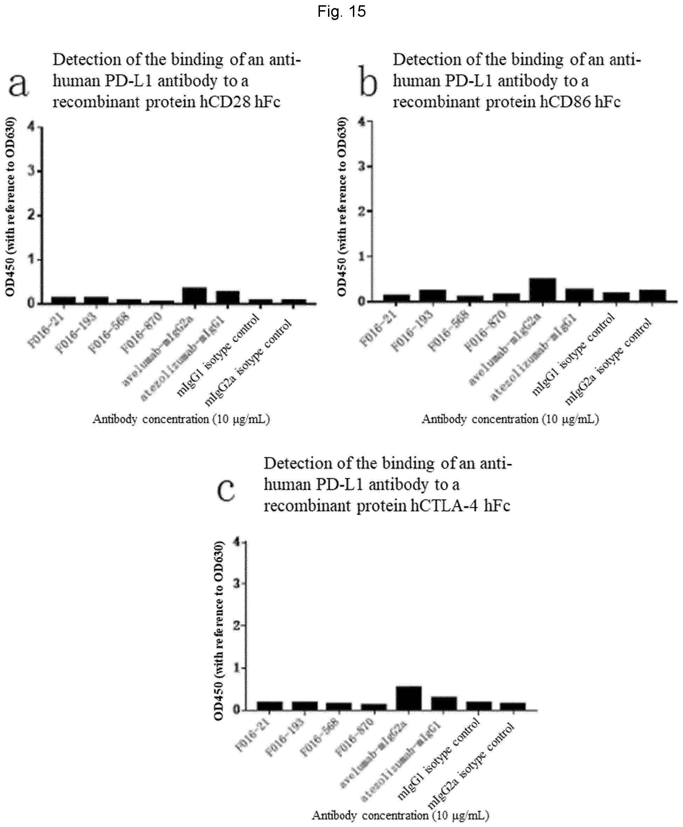

Results of the binding specificity of the anti-human PD-L1 antibody to recombinant proteins hPD-L1 hFc, hPD-L2 hFc, hICOSLGhFc and hB7-H3 hFc were shown in Fig. 14; and results of the binding specificity of the anti-human PD-L1 antibody to recombinant proteins hCD28 hFc, hCD86 hFc and hCTLA-4 hFc were shown in Fig. 15. Results showed that, 4 antibodies all did not bind to the B7 family of proteins other than the PD-L1 and other related proteins (hPD-L1 hFc, hPD-L2 hFc, hICOSLGhFc, hB7-H3 hFc, hCD28 hFc, hCD86 hFc and hCTLA-4 hFc); and in addition to binding to the PD-L1, the control antibodies had relatively high binding to PD-L2, ICOSLG, B7-H3, CD28, CD86 and CTLA-4. Therefore, it is indicated that, compared with the control antibodies, the binding specificity of the anti-human PD-L1 antibody of the application was stronger.

### Embodiment 7

### In Vivo Activity Characterization of Anti-human PD-L1 antibody

In this embodiment, the effect of the anti-human PD-L1 antibody on blocking a mouse tumor model was studied. Since the anti-human PD-L1 mouse monoclonal antibody obtained in the application did not cross with a mouse source PD-L1, HPD-L1 transgenic mice on a C57BL/6 background (Biocytogen) were used. An experimental method included the following.

Mice were subcutaneously inoculated with 1×10E6 MC38 hPD-L1 stably expressing colorectal cancer cells on the right side. The day of inoculation was defined as Day 0 of study. When an average tumor volume reached 60-80mm³, the mice were randomly grouped according to the tumor volume; and at Day 8 of study, the mice were randomly divided into 10 groups according to the tumor volume, each group had 8 mice, and intraperitoneal administration was started. A dosage regimen was shown in Table 4.

**Table 4**

| Group | Dosage (mg/kg) | Administration volume (µL/g) | Administration frequency |
|---|---|---|---|
| DPBS | \ | 10 | Q3D*6 |
| mlgG2a isotype control | 10 | 10 | Q3D*6 |
| Avelumab-mlgG2a | 10 | 10 | Q3D*6 |
| F016-21-mlgG2a | 10 | 10 | Q3D*6 |
| F016-870-mlgG2a | 10 | 10 | Q3D*6 |
| F016-568-mlgG2a | 10 | 10 | Q3D*6 |
| Atezolizumab-mlgG1 | 10 | 10 | Q3D*6 |
| F016-21-mlgG1 | 10 | 10 | Q3D*6 |
| F016-870-mlgG1 | 10 | 10 | Q3D*6 |
| F016-568-mlgG1 | 10 | 10 | Q3D*6 |

The tumor volume was measured and recorded from Day 8 onwards; and long and short diameters of the tumor were measured with a vernier caliper twice per week within a research duration. The tumor volume was calculated according to (1/2)×long diameter× (short diameter)². When the sizes of the mice decreased by 20% or the tumor volume exceeded 2000mm³, a tumor humane endpoint reached, and the mice were killed with a CO₂ suffocation method.

**Table 5**

| Study on Anti-PD-L1 Antibody Reducing Tumor Volume (mm³) of hPD-L1 Transgenic Mouse MC38-hPD-L1 Colorectal Cancer | | | | | |
|---|---|---|---|---|---|
| Days after implantation | mlgG2a Isotype control | Avelumab-mlgG2a | F016-21-mlgG2a | F016-870-mlgG2a | F016-568-mlgG2a |
| 8 | 81.22 | 78.95 | 76.47 | 78.08 | 78.30 |
| 13 | 180.09 | 47.78 | 27.48 | 21.61 | 43.98 |
| 16 | 313.49 | 16.62 | 17.00 | 8.78 | 57.50 |
| 20 | 527.16 | 41.96 | 8.83 | 8.73 | 85.32 |
| 23 | 782.96 | 61.03 | 23.97 | 5.45 | 138.10 |
| 27 | 1157.99 | 103.26 | 35.16 | 5.42 | 157.97 |
| 30 | 1377.41 | 92.89 | 49.02 | 6.87 | 182.92 |
| 35 | \ | 134.49 | 119.71 | 3.72 | 187.77 |
| 37 | \ | 202.65 | 127.81 | 6.16 | 196.79 |
| 41 | \ | 217.59 | 217.97 | 6.91 | 239.52 |
| 44 | \ | 380.00 | 231.12 | 4.49 | 246.06 |
| 8 | 88.17 | 78.44 | 78.20 | 81.98 | 77.04 |
| 13 | 128.48 | 125.34 | 81.36 | 131.67 | 142.72 |
| 16 | 258.78 | 218.09 | 116.86 | 258.33 | 203.41 |
| 20 | 447.24 | 356.18 | 201.72 | 437.60 | 297.52 |
| 23 | 747.28 | 499.64 | 310.36 | 615.61 | 431.16 |
| 27 | 1158.65 | 765.84 | 410.65 | 809.91 | 581.72 |
| 30 | 1516.76 | 1043.87 | 668.36 | 1151.84 | 750.87 |
| 35 | 2064.17 | 1687.19 | 855.84 | 1654.36 | 1057.45 |

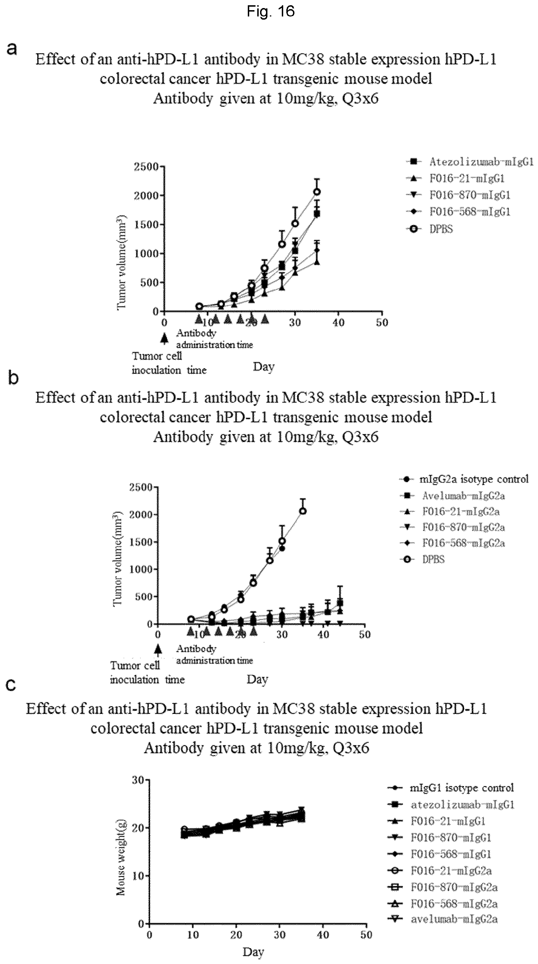

Characterization results of the in vivo activity of the anti-human PD-L1 antibody were shown in Table 5 and Fig. 16. Results showed that, the antitumor activity of the mlgG1 subtype anti-hPD-L1 antibodies F016-21 (TGI=69.19%, CR:3/8) and F016-870 (TGI=53.22%, CR:0/8) was superior to that of Atezolizumab-mlgG1 (TGI=36.29%, CR:0/8) (as shown in Fig. 16a). The antitumor activity of the mlgG2a subtype anti-hPD-L1 antibodies F016-21, F016-870 and F016-568 was significant; and TGI was respectively 103.83%, 106.73% and 92.62%. The complete remission (CR) in the 8 mice was 7, 7 and 4 (that is, CR being 7/8, 7/8 and 4/8, respectively) (as shown in Fig. 16b). In addition, each administration group of the anti-hPD-L1 antibody had no significant effect on the weights of the mice (as shown in Fig. 16c).

The above embodiments are preferred embodiments of the application, but the embodiments of the application are not limited by the above embodiments, and any other changes, modifications, substitutions, combinations, and simplifications that do not deviate from the spirit and principle of the application should be equivalent substitutions and are included within the protection scope of the application.

## Claims

1. An anti-PD-L1 antibody or an antigen-binding fragment thereof, wherein the antibody comprises the following Complementarity-Determining Regions (CDRs):
an HCDR1 as shown in an amino acid sequence, which is shown as any one of SEQ ID NO.19, 25, 31 or 37 or has at least 95% identity to a sequence as shown in any one of SEQ ID NO.19, 25, 31 or 37; an HCDR2 as shown in an amino acid sequence, which is shown as any one of SEQ ID NO.20, 26, 32 or 38 or has at least 95% identity to a sequence as shown in any one of SEQ ID NO.20, 26, 32 or 38; an HCDR3 as shown in an amino acid sequence, which is shown as any one of SEQ ID NO.21, 27, 33 or 39 or has at least 95% identity to a sequence as shown in any one of SEQ ID NO.21, 27, 33 or 39;
an LCDR1 as shown in an amino acid sequence, which is shown as any one of SEQ ID NO.22, 28, 34 or 40 or has at least 95% identity to a sequence as shown in any one of SEQ ID NO.22, 28, 34 or 40; an LCDR2 as shown in an amino acid sequence, which is shown as any one of SEQ ID NO.23, 29, 35 or 41 or has at least 95% identity to a sequence as shown in any one of SEQ ID NO.23, 29, 35 or 41; and an LCDR3 as shown in an amino acid sequence, which is shown as any one of SEQ ID NO.24, 30, 36 or 42 or has at least 95% identity to a sequence as shown in any one of SEQ ID NO.24, 30, 36 or 42.

2. The anti-PD-L1 antibody or the antigen-binding fragment thereof according to claim 1, wherein the antibody comprises a heavy chain variable region and a light chain variable region; the amino acid sequence of the heavy chain variable region is shown in any one of SEQ ID NO: 5, 9, 13 or 17 or has at least 95% identity to a sequence as shown in any one of SEQ ID NO: 5, 9, 13 or 17; and
the amino acid sequence of the light chain variable region is shown in any one of SEQ ID NO: 6, 10, 14 or 18 or has at least 95% identity to a sequence as shown in any one of SEQ ID NO: 6, 10, 14 or 18.

3. The anti-PD-L1 antibody or the antigen-binding fragment thereof according to claim 1, wherein the antibody has a constant region; the heavy chain constant region is selected from any one of IgG1, IgG2, IgG3, IgG4, IgA, IgD, IgE or IgM; and the light chain constant region is a κ chain or a λ chain.

4. The anti-PD-L1 antibody or the antigen-binding fragment thereof according to any one of claims 1 to 3, wherein the antibody is any one or several of a bispecific antibody, a CDR grafted antibody or a polymer antibody; and the antigen-binding fragment is any one or several of scFv, Fab, Fab', F (ab')2, Fv, and a single domain antibody.

5. A nucleic acid encoding a sequence of the anti-PD-L1 antibody or the antigen-binding fragment thereof according to any one of claims 1 to 4, wherein
optionally, the nucleic acid comprises: a first nucleic acid encoding a heavy chain variable region of the antibody or the antigen-binding fragment thereof, and/or a second nucleic acid encoding a light chain variable region of the antibody or the antigen-binding fragment thereof.

6. The nucleic acid according to claim 5, wherein a base sequence of the first nucleic acid is shown in any one of SEQ ID NO:3, SEQ ID NO:7, SEQ ID NO:11 or SEQ ID NO:15 or has at least 95% identity to a sequence as shown in any one of SEQ ID NO:3, SEQ ID NO:7, SEQ ID NO:11 or SEQ ID NO:15; and
a base sequence of the second nucleic acid is shown in any one of SEQ ID NO: 4, SEQ ID NO: 8, SEQ ID NO: 12 or SEQ ID NO: 16 or has at least 95% identity to a sequence as shown in any one of SEQ ID NO: 4, SEQ ID NO: 8, SEQ ID NO: 12 or SEQ ID NO: 16.

7. A vector comprising the nucleic acid according to claim 5 or 6.

8. A cell comprising the nucleic acid according to claim 5 or 6, or the vector according to claim 7.

9. A pharmaceutical composition comprising the anti-PD-L1 antibody or the antigen-binding fragment thereof according to any one of claims 1 to 4, the nucleic acid according to claim 5 or 6, the vector according to claim 7 or the cell according to claim 8.

10. The applications of the anti-PD-L1 antibody or the antigen-binding fragment thereof according to any one of claims 1 to 4, the nucleic acid according to claim 5 or 6, the vector according to claim 7, the cell according to claim 8 or the pharmaceutical composition according to claim 9 in preparation of drugs for treating PD-L1-mediated diseases or symptoms.
